(19) Europäisches Patentamt
European Patent Office
Office européen des brevets

(11) **EP 3 312 598 B1**

(12) **EUROPÄISCHE PATENTSCHRIFT**

(45) Veröffentlichungstag und Bekanntmachung des Hinweises auf die Patenterteilung:
**04.08.2021 Patentblatt 2021/31**

(51) Int Cl.:
***G01N 23/16*** (2018.01)     ***G01N 33/38*** (2006.01)

(21) Anmeldenummer: **17197301.9**

(22) Anmeldetag: **19.10.2017**

(54) **VORRICHTUNG ZUM ÜBERWACHEN EINES WENIGSTENS EINEN OFEN VERWENDENDEN HERSTELLUNGSVERFAHRENS VON GIPSERZEUGNISSEN**

DEVICE FOR MONITORING A PRODUCTION PROCESS FOR GYPSUM PRODUCTS USING AT LEAST ONE FURNACE

DISPOSITIF DE SURVEILLANCE D'AU MOINS UN PROCÉDÉ DE FABRICATION DES ÉLÉMENTS EN PLÂTRE UTILISANT UN FOUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.10.2016 DE 102016012670**

(43) Veröffentlichungstag der Anmeldung:
**25.04.2018 Patentblatt 2018/17**

(73) Patentinhaber: **Fagus-GreCon Greten GmbH & Co. KG**
**31061 Alfeld (DE)**

(72) Erfinder: **BERGMANN, Bernd**
**30900 Wedemark (DE)**

(74) Vertreter: **Jabbusch, Matthias**
**Jabbusch Siekmann & Wasiljeff**
**Patentanwälte**
**Roscherstrasse 12**
**30161 Hannover (DE)**

(56) Entgegenhaltungen:
DE-C- 836 436     DE-U1- 29 706 475
US-A- 3 260 642   US-A- 4 599 514

**Beschreibung**

Die Erfindung betrifft eine Vorrichtung zum Überwachen eines wenigstens einen Ofen verwendenden Herstellungsverfahrens von Gipserzeugnissen, umfassend eine Vorrichtung zum Messen von Flächenmassebildern in einem Nassbereich vor Eintritt der Gipserzeugnisse in den Ofen. Die Erfindung betrifft des Weiteren ein Verfahren zum Überwachen eines wenigstens einen Ofen verwendenden Herstellungsverfahrens von Gipserzeugnissen, bei dem im Nassbereich vor Eintritt der Gipserzeugnisse die Flächenmassen der Gipserzeugnisse gemessen werden und dabei ein Flächenmassebild aufgenommen wird, insbesondere unter Einsatz einer vorgenannten Vorrichtung.

Bei den vorgenannten Gipserzeugnissen kann es sich insbesondere um Gipskartonerzeugnisse handeln. Diese werden im Trockenausbau in Wohngebäuden oder auch zu anderen Zwecken eingesetzt.

Zwischen zwei Kartonlagen ist eine Gipslage zu bringen. So wird für eine Gipskartonplatte auf 18kg Gips-Flächenmasse 5kg Wasser gegeben, damit die Gipsmasse fließfähig ist und zwischen die Kartonlagen gebracht werden kann. Im Ofen ist das Wasser wieder aus der Gipsmasse herauszutrocknen.

Es ist bekannt, im Nassbereich vor Eintritt der Gipserzeugnisse in den Ofen eine Vorrichtung zum Messen von Flächenmassebildern anzuordnen. Diese Vorrichtung ist vorzugsweise eine Röntgenmesseinrichtung, der ein Detektor zugeordnet ist. Mit der Vorrichtung kann dann die Intensität der Aufnahme der Röntgenstrahlung durch die Gipserzeugnisse gemessen werden. Eine am Detektor gemessene Intensität kann nach folgender Formel in Flächenmasse umgerechnet werden:

$$I(f_{nass}) = I_0 e^{-\mu_{nass} f_{nass}} = I_0 e^{-\mu_{Gips} f_{Gips}} e^{-\mu_{Wasser} f_{Wasser}}$$

Für diese und nachfolgende Betrachtungen wird das Mischungsverhältnis zwischen Gipsmasse und Wassermasse als konstant beziehungsweise vorgegeben vorausgesetzt:

$$k_{\frac{W}{G}} = \frac{m_{Wasser}}{m_{Gips}} = \frac{f_{Wasser}}{f_{Gips}}$$

Für die Flächenmasse der Gipserzeugnisse gilt vor dem Ofen:

$$f_{nass} = f_{Gips} + f_{Wasser}$$

Im Stand der Technik ist zur Bestimmung des Restfeuchtegehaltes an Wasser in den Gipserzeugnissen nach Austritt aus dem Ofen bisher so vorgegangen worden, dass ein Probegipserzeugnis aus dem Produktionsprozess herausgenommen wurde und getrocknet wurde. Dieses wurde dann erneut mit der Vorrichtung im Nassbereich vermessen.

Zur Bestimmung der Flächenmasse $f_{nass}$ wird also im Stand der Technik eine Platte entnommen, vermessen und gewogen. Diese Messwerte werden nun zur Ermittlung der Absorptionskoeffizienten verwendet. Hierzu werden die Daten der Messung der Vorrichtung verfolgt, um die mittlere Intensität der Platte $I_{nass_{Platte}}$ zu bestimmen:

$$I_{nass_{Platte}} = I_0 e^{-\mu_{nass} f_{nass_{Platte}}}$$

Dieselbe Platte wird anschließend im Ofen vollständig getrocknet und danach gewogen:

$$f_{trocken} = f_{Gips}$$

Nun wird die Platte im trockenen Zustand erneut durch die Vorrichtung zum Messen von Flächenmassebildern gefahren, um die mittlere Intensität $I_{trocken_{Platte}}$ der trockenen Platte zu bestimmen. Aus diesen beiden Werten wird der Absorptionsfaktor $\mu_{Gips}$ des Gipses wie folgt bestimmt:

$$I_{trocken_{Platte}} = I_0 e^{-\mu_{Gips} f_{trocken_{Platte}}}$$

$$\mu_{Gips} = \frac{1}{f_{trocken_{Platte}}} \ln\left(\frac{I_0}{I_{trocken_{Platte}}}\right)$$

Damit kann nun auch der Absorptionsfaktor des Wassers bestimmt werden:

$$I_{nass_{Platte}} = I_0 e^{-\mu_{nass} f_{nass_{Platte}}}$$

$$I_{nass_{Platte}} = I_0 e^{-\mu_{Gips} f_{Gips_{Platte}}} e^{-\mu_{Wasser} f_{Wasser_{Platte}}}$$

$$I_{nass_{Platte}} = I_{trocken_{Platte}} e^{-\mu_{Wasser} f_{Wasser_{Platte}}}$$

$$\mu_{Wasser} = \frac{1}{f_{Wasser_{Platte}}} \ln\left(\frac{I_{trocken_{Platte}}}{I_{nass_{Platte}}}\right)$$

Mit Hilfe der Werte von $\mu_{Gips}$, $\mu_{Wasser}$ und $\frac{k_W}{G}$ kann nun die Flächenmasse des Gipses gemessen werden:

$$f_{Wasser} = k_{\frac{W}{G}} f_{Gips}$$

$$I_{nass_{Platte}} = I_0 e^{-\mu_{Gips} f_{Gips_{Platte}}} e^{-\mu_{Wasser} f_{Wasser_{Platte}}}$$

$$I_{nass_{Platte}} = I_0 e^{-\mu_{Gips} f_{Gips_{Platte}}} e^{-\mu_{Wasser} k_{\frac{W}{G}} f_{Gips}}$$

$$I_{nass_{Platte}} = I_0 e^{-\left(\mu_{Gips} f_{Gips_{Platte}} + \mu_{Wasser} k_{\frac{W}{G}} f_{Gips}\right)}$$

$$\frac{I_0}{I_{nass_{Platte}}} = e^{f_{Gips_{Platte}}\left(\mu_{Gips} + \mu_{Wasser} k_{\frac{W}{G}}\right)}$$

$$f_{Gips_{Platte}} = \frac{1}{\mu_{Gips} + \mu_{Wasser}\frac{k_W}{G}} \ln\left(\frac{I_0}{I_{nass_{Platte}}}\right)$$

Auf diese Weise gelang ein Einrichten der bekannten Vorrichtung zum Überwachen des Herstellungsverfahrens auf die Messung. Für jede Platte, die aus einem Endlosstrang geschnitten wird, konnte die Flächenmasse des Trockengipses ermittelt werden.

Das Herausnehmen einer Platte aus dem Produktionsprozess und ihre gegebenenfalls separate Trocknung und Vermessung sind jedoch aufwendig.

Eine Vorrichtung der eingangs genannten Gattung ist in der DE 836 436 C genannt. Dort ist bereits im Nassbereich vor Eintritt von Gipserzeugnissen in einen Ofen eine Vorrichtung zum Messen der Flächenmasse beschrieben. Die US 326642 zeigt Vorrichtungen zum Messen der Flächenmasse vor und nach einer Trocknungseinrichtung, diese sind über einen Computer zur Bestimmung der Feuchte miteinander verbunden. Die DE 297 06 475 U1 beschreibt eine Vorrichtung zur Messung des Flächengewichts eines Stoffgemisches mit einer Strahlungsquelle, deren Strahlung durch das Stoffgemisch hindurch auf eine Detektorvorrichtung sendbar ist.

Der Erfindung liegt die Aufgabe zugrunde, eine während des laufenden Produktionsprozess einzusetzende Überwachungsvorrichtung aufzuzeigen, mit der eine Bestimmung der Flächenmassen der Gipserzeugnisse sowie eine Bestimmung des Restfeuchtegehaltes ermöglicht ist. Darüber hinaus soll ein Verfahren zum Überwachen eines wenigstens einen Ofen verwendenden Herstellungsverfahrens von Gipserzeugnissen aufgezeigt werden.

Diese Aufgabe ist hinsichtlich der Vorrichtung erfindungsgemäß dadurch gelöst, dass in einem Trockenbereich nach Austritt der Gipserzeugnisse aus dem Ofen zumindest eine zweite Vorrichtung zum Messen eines weiteren Flächenmassebildes angeordnet ist und beide Vorrichtungen miteinander verknüpft sind, so dass beide Flächenmassebilder zum Zuordnen der beiden Messungen zu ein und demselben Gipserzeugnis übereinander legbar sind, wobei jede Vorrichtung eine Röntgenmesseinrichtung (5, 6, 6') ist.

Mit der zweiten Vorrichtung zum Messen von Flächenmassebildern lässt sich jedes den Ofen verlassende Gipserzeugnis vollständig oder in einem definierten Teilbereich vermessen. Durch die Verknüpfung beider Vorrichtungen werden die Messdaten der Messeinrichtungen an den einzelnen Positionen platten- oder abschnittsbezogen weitergeleitet. Die an der Vorrichtung nach dem Ofen gemessene Intensität der Platte mit Restfeuchte ermöglicht direkt die Bestimmung des Feuchtegehaltes:

$$f_{Restfeuchte_{Platte}} = f_{Gips_{Platte}} + f_{Wasser_{Platte}}$$

$$I_{Restfeuchte_{Platte}} = I_0 e^{-\mu_{Gips} f_{Gips_{Platte}}} e^{-\mu_{Wasser} f_{Wasser_{Platte}}}$$

$$\frac{I_0}{I_{Restfeuchte_{Platte}}} = e^{\left(\mu_{Gips} f_{Gips_{Platte}} + \mu_{Wasser} f_{Wasser_{Platte}}\right)}$$

$$\mu_{Gips} f_{Gips_{Platte}} + \mu_{Wasser} f_{Wasser_{Platte}} = \ln\left(\frac{I_0}{I_{Restfeuchte_{Platte}}}\right)$$

$$f_{Wasser_{Platte}} = \frac{1}{\mu_{Wasser}} \ln\left(\frac{I_0}{I_{Restfeuchte_{Platte}}}\right) - \frac{\mu_{Gips}}{\mu_{Wasser}} f_{Gips_{Platte}}$$

$$h_{Restfeuchte_{ATRO}} = \frac{f_{Wasser_{Platte}}}{f_{Gips_{Platte}}}$$

$$h_{Restfeuchte_{ATRO}} = \frac{\frac{1}{\mu_{Wasser}} \ln\left(\frac{I_0}{I_{Restfeuchte_{Platte}}}\right)}{\frac{1}{\mu_{Gips} + \mu_{Wasser}\frac{k_W}{G}} \ln\left(\frac{I_0}{I_{nass_{Platte}}}\right)} - \frac{\mu_{Gips}}{\mu_{Wasser}}$$

Das Herausnehmen von Probestücken und das gegebenenfalls manuelle Wiegen ist vorteilhaft nicht mehr notwendig. Die Vermessung erfolgt schnell und während des laufenden Produktionsprozess.

Eine Waage kann gegebenenfalls noch für Kalibrierzwecke vorgesehen sein.

Nach der Erfindung ist vorgesehen, dass jede Vorrichtung zum Messen von Flächenmassebildern eine Röntgenmesseinrichtung ist. Mit Röntgenmesseinrichtungen können Intensitäten bestimmt werden.

Die verfahrensseitige Lösung der Aufgabe ist dadurch gekennzeichnet, dass in einem Trockenbereich nach Austritt der Gipserzeugnisse aus dem Ofen die Flächenmasse der Gipserzeugnisse erneut gemessen wird und dass beide gemessenen Flächenmassebilder zum Zuordnen der beiden Messungen zu ein und demselben Gipserzeugnis übereinandergelegt werden.

Das erfindungsgemäße Verfahren ermöglicht ein richtiges Zuordnen der gemessenen Flächenmassebilder zueinander. In einer Fertigungsstrecke für Gipserzeugnisse können die beiden Flächenmassemessungen voneinander räumlich weit entfernt sein, beispielsweise über 100 m. Nach dem Verfahren gelingt es gleichwohl, ausschließlich anhand der aufgenommenen Bilder eine richtige Zuordnung beider Bilder zu ein und demselben Gipserzeugnis zu erreichen. Dies ist darin begründet, dass die Flächenmasseverteilung in den Gipserzeugnissen markant und für jedes Gipserzeugnis eigen ist. Es liegen charakteristische Innenhomogenitäten vor.

Aufgrund der Charakteristik der Innenhomogenitäten ist es nach einer Weiterbildung des erfindungsgemäßen Verfahrens nicht notwendig, stets die Flächenmasse der gesamten Gipserzeugnisse zu messen. Ausreichend ist vielmehr das jedes Flächenmassebild einen Ausschnitt des Gipserzeugnisses darstellt. Dieser Ausschnitt kann beispielsweise etwa 10 cm breit sein, bereits bei einem derartigen Flächenmasseausschnitt eines Gipserzeugnisses ergeben sich charakteristische Merkmale für ein und nur ein Gipserzeugnis.

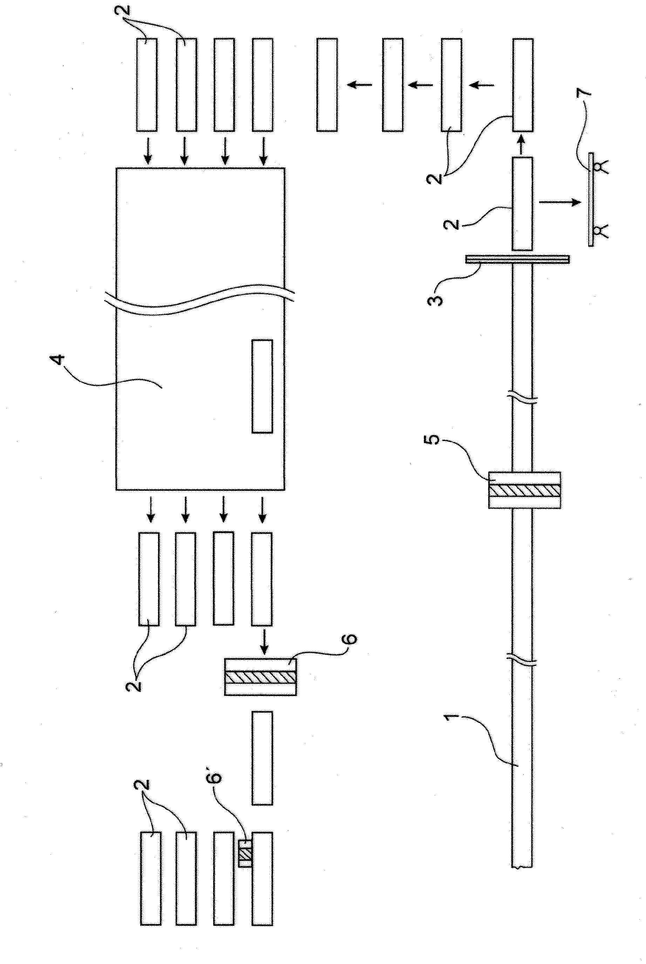

Ein Ausführungsbeispiel der Erfindung, aus dem sich weitere erfinderische Merkmale ergeben, ist in der Zeichnung dargestellt.

Die Zeichnung zeigt in schematischer Weise den Produktionsprozess für Gipserzeugnisse unter Verwendung einer erfindungsgemäßen Vorrichtung.

Die Gipserzeugnisse in der Figur werden aus einem Endlosstrang 1 hergestellt. Die Gipserzeugnisse sind Gipskartonplatten 2, die aus dem Endlosstrang 1 durch Abteilen mit einer Schlagschere 3 abgeteilt werden. Im weiteren Verlauf werden mehrere Gipskartonplatten 2 in einen Ofen 4 eingestellt, in dem sie getrocknet werden. Getrocknete Gipskartonplatten 2 verlassen den Ofen und werden aufgestapelt.

Bei der Herstellung der Gipskartonplatten 2 wird im Bereich des Endlosstrangs 1 häufig zu viel Wasser aufgegeben. Gewünscht ist eine gute Fließfähigkeit, das Wasser ist jedoch anschließend mit Energieeinsatz im Ofen aus den Gipskartonplatten 2 herauszutrocknen.

Die erfindungsgemäße Vorrichtung zum Überwachen des Herstellungsverfahrens umfasst zwei Röntgenmesseinrichtungen 5 und 6. Mit der Röntgenmesseinrichtung 5 werden Abschnitte des Endlosstrangs 1 hinsichtlich der Flächenmasse aus Gips und Wasser vermessen. Die aus den entsprechenden Abschnitten des Endlosstrangs 1 gefertigten Gipskartonplatten 2 werden nach Austritt aus dem Ofen 4 mit der Röntgenmesseinrichtung 6 noch einmal gemessen. Mit der Röntgenmesseinrichtung 6 wird der Restfeuchtegehalt in den Gipskartonplatten 2 überprüft, fällt dieser zu hoch aus, kann im Bereich des Endlosstrangs 1 weniger Wasser aufgegeben werden.

Mit der Röntgenmesseinrichtung 6 kann eine vollständige Messung jeder Gipskartonplatte 2 erfolgen, mit einer alternativen Röntgenmesseinrichtung 6' ist ein definierter Abschnitt jeder Gipskartonplatte 2 messbar.

Eine noch vorhandene Waage 7 kann für Kalibrierzwecke verwendet werden.

## Patentansprüche

1. Vorrichtung zum Überwachen eines wenigstens einen Ofen verwendenden Herstellungsverfahrens von Gipserzeugnissen, umfassend eine Vorrichtung zum Messen von Flächenmassebildern in einem Nassbereich vor Eintritt der Gipserzeugnisse in den Ofen,

   **dadurch gekennzeichnet,**

   **dass** in einem Trockenbereich nach Austritt der Gipserzeugnisse (2) aus dem Ofen (4) zumindest eine zweite Vorrichtung zum Messen eines weiteren Flächenmassebildes angeordnet ist und beide Vorrichtungen miteinander verknüpft sind, so dass beide Flächenmassebilder zum Zuordnen der beiden Messungen zu ein und demselben Gipserzeugnis (2) übereinander legbar sind, wobei jede Vorrichtung eine Röntgenmesseinrichtung (5, 6, 6') ist.

**2.** Vorrichtung nach Anspruch 1, **dadurch gekennzeichnet, dass** den Vorrichtungen zum Messen von Flächenmassebildern zumindest eine Waage (7) als Kalibrierwerkzeug zugeordnet ist.

**3.** Verfahren zum Überwachen eines wenigstens einen Ofen verwendenden Herstellungsverfahrens von Gipserzeugnissen, bei dem im Nassbereich vor Eintritt der Gipserzeugnisse die Flächenmassen der Gipserzeugnisse gemessen werden und dabei ein Flächenmassebild aufgenommen wird, insbesondere unter Einsatz einer Vorrichtung nach einem der Ansprüche 1 bis 2,
**dadurch gekennzeichnet,**
**dass** in einem Trockenbereich nach Austritt der Gipserzeugnisse (2) aus dem Ofen (4) die Flächenmasse der Gipserzeugnisse (2) erneut gemessen wird und dass beide gemessenen Flächenmassebilder zum Zuordnen der beiden Messungen zu ein und demselben Gipserzeugnis (2) übereinandergelegt werden.

**4.** Verfahren nach Anspruch 3, **dadurch gekennzeichnet, dass** jedes Flächenmassebild einen Ausschnitt des Gipserzeugnisses (2) darstellt.

**5.** Verfahren nach Anspruch 4, **dadurch gekennzeichnet, dass** eine Ausschnittsbreite von etwa 10 cm eingestellt wird.

**Claims**

**1.** A device for monitoring a production process for gypsum products using at least one furnace, comprising a device for measuring area density images in a wet region prior to the entry of the gypsum products into the furnace,
**characterised in that**
at least one second device for measuring a further area density image is arranged in a dry region following the exit of the gypsum products (2) from the furnace (4), and both devices are linked to one another so that both area density images can be placed one above the other to assign the two measurements to one and the same gypsum product (2), wherein each device is an X-ray measuring device (5, 6, 6').

**2.** The device according to claim 1, **characterised in that** at least one scale (7) is assigned as a calibration tool to the devices for measuring area density images.

**3.** A method for monitoring a production process for gypsum products using at least one furnace, in which method the area densities of the gypsum products are measured in the wet region prior to the entry of the gypsum products and in so doing an area density image is recorded, in particular with use of a device according to one of claims 1 to 2,
**characterised in that**
the area density of the gypsum products (2) is measured again in a dry region following the exit of the gypsum products (2) from the furnace (4), and **in that** both measured area density images are placed one above the other to assign the two measurements to one and the same gypsum product (2).

**4.** The method according to claim 3, **characterised in that** each area density image shows a portion of the gypsum product (2).

**5.** The method according to claim 4, **characterised in that** a portion width of approximately 10 cm is set.

**Revendications**

**1.** Dispositif, destiné à superviser un procédé de fabrication de produits à base de plâtre, utilisant au moins un four, comprenant un dispositif de mesure de clichés de masse surfacique dans une zone humide, avant l'entrée des produits à base de plâtre dans le four,
**caractérisé**
**en ce qu'**au moins un deuxième dispositif de mesure d'un autre cliché de masse surfacique est placé dans une zone sèche après la sortie des produits à base de plâtre (2) du four (4) et les deux dispositifs sont connectés l'un à l'autre, de sorte que les deux clichés de masse surfacique sont superposables, pour l'attribution des deux mesures à un seul et même produit à base de plâtre (2),
chaque dispositif étant un système de mesure par rayon X (5, 6, 6').

**2.** Dispositif selon la revendication 1, **caractérisé en ce qu'**aux dispositifs de mesure de clichés de masse surfacique est attribuée au moins une balance (7), en tant qu'outil de calibrage.

**3.** Procédé, destiné à superviser un procédé de fabrication de produits à base de plâtre, utilisant au moins un four, lors duquel dans la zone humide avant l'entrée des produits à base de plâtre dans le four, l'on mesure les masses surfaciques des produits à base de plâtre et on prend à cet effet un cliché de masse surfacique, notamment en utilisant un dispositif selon l'une quelconque des revendications 1 et 2, **caractérisé en ce que** l'on mesure une nouvelle fois les produits à base de plâtre (2) dans une zone sèche, après la sortie des produits à base de plâtre (2) du four (4) et **en ce que** l'on superpose les deux clichés de masse surfacique mesurés, pour attribuer les deux mesures à un seul et même produit à base de plâtre (2).

**4.** Produit selon la revendication 3, **caractérisé en ce que** chaque cliché de masse surfacique représente un extrait des produits à base de plâtre (2).

**5.** Procédé selon la revendication 4, **caractérisé en ce que** l'on règle une largeur d'extrait d'environ 10 cm.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 836436 C
- US 326642 A
- DE 29706475 U1